# EUROPEAN PATENT SPECIFICATION

(11) **EP 4 009 876 B1**
(45) Date of publication and mention of the grant of the patent: **07.01.2026**
(21) Application number: 20750641.1
(22) Date of filing: 31.07.2020
(51) Int. Cl.: A61B 8/08, A61B 8/06, A61B 8/00

(54) **CONTRAST ENHANCED ULTRASOUND IMAGING WITH CHANGING SYSTEM OPERATION DURING WASH-IN, WASH-OUT**
KONTRASTVERSTÄRKTE ULTRASCHALLBILDGEBUNG MIT VERÄNDERBAREM SYSTEMBETRIEB WÄHREND DES EIN- UND AUSWASCHENS
IMAGERIE ULTRASONORE À CONTRASTE AMÉLIORÉ AVEC FONCTIONNEMENT DU SYSTÈME MODIFIABLE PENDANT LE PASSAGE DANS ET HORS D'UNE ZONE

(30) Priority: 05.08.2019 US 201962882644 P
(43) Date of publication of application: 15.06.2022
(73) Proprietor: Koninklijke Philips N.V., 5656 AG Eindhoven (NL)
(72) Inventor: HOPE SIMPSON, David, 5656 AE Eindhoven (NL); BAE, Unmin, 5656 AE Eindhoven (NL); SHAMDASANI, Vijay, Thakur, 5656 AE Eindhoven (NL); POWERS, Jeffry, Earl, 5656 AE Eindhoven (NL); LOUPAS, Thanasis, 5656 AE Eindhoven (NL)
(86) International application number: PCT/EP2020/071644
(87) International publication number: WO 2021/023651

(56) References cited:
- EP-A1- 2 394 580
- US-A1- 2009 124 907
- US-A1- 2010 172 562
- LUECK G J ET AL: "Hepatic Perfusion Imaging Using Factor Analysis of Contrast Enhanced Ultrasound", IEEE TRANSACTIONS ON MEDICAL IMAGING, IEEE SERVICE CENTER, PISCATAWAY, NJ, US, vol. 27, no. 10, 1 October 2008 (2008-10-01), pages 1449 - 1457, XP011226135, ISSN: 0278-0062, DOI: 10.1109/TMI.2008.922695

## Description

This invention relates to medical diagnostic ultrasound systems and, in particular, to ultrasonic imaging of contrast agent wash-in and wash-out with changing ultrasound system operation.

The use of ultrasound to image blood flow to suspected cancerous pathology is often aided by the use of contrast agents. An ultrasonic contrast agent is a solution of microbubbles which is infused into the bloodstream. The microbubbles are highly echogenic, returning strong echo signals which are easily detected and highlight regions of blood flow. Furthermore, the echo signals from microbubbles contain significant harmonic frequency content, enabling the microbubble echo signals to be readily segmented from signals returning from tissue. Following the commencement of contrast agent infusion, often in the form of a bolus injection, the contrast agent in the bloodstream begins to arrive at a region of interest (ROI) in the body and is readily discernable as the continuing arrival of the contrast agent increases the concentration of the agent in the ROI, first in the larger, faster-flowing vessels and then in the microvasculature of the surrounding tissue. This is the so-called "wash-in" phase of the contrast imaging procedure. Thereafter, the concentration of the contrast agent declines as the bolus of contrast agent passes through the ROI and is filtered out by the lungs. This is the so-called "wash-out" phase of the procedure. By observing and measuring the times of the phases and the intensity of the contrast agent build-up, clinicians are able to distinguish blood flow characteristics of cancerous and normal tissues.

Cancerous lesions are often characterized by the development of blood vessels which feed the cancerous lesions. The functioning of these blood vessels is marked by relatively significant blood flows and the early arrival of the contrast agent during the wash-in phase. Thereafter, the contrast agent will light up the surrounding parenchyma as the contrast agent begins to decline in the larger vessels during the wash-out phase. It would be desirable to be able to control the ultrasound system to use signal and image processing changes which are specifically tailored to enhance the detection of these different contrast agent behaviors during different phases of a contrast agent imaging procedure.

One of the main regions of the body which benefits from contrast agent image enhancement is the liver. Hepatitis B and hepatitis C patients have been found to be at an increased risk of developing primary liver cancer, hepatocellular carcinoma (HCC). Due to the discovery that hepatitis C was being contracted by patients through blood transfusions in the early 1980's, there remain a significant number of hepatitis C patients who need to be examined regularly for the onset of HCC, as the lesions are best treated in their early stages. The usual progression of the disease is from hepatitis to liver cirrhosis to HCC. An easy-to-use monitoring technique for liver disease progression would have widespread application in assisting in the early detection of this serious disease.

Since liver lesions, like other cancers, are most effectively treated when detected early, high-risk patients should be monitored frequently for signs of these diseases. But in their early stages liver lesions are often difficult to detect through conventional diagnostic imaging due to their small size. Thus, clinicians often conduct their diagnoses to look for other signs that a lesion is developing. One of these signs is change in the blood flow to the liver. The liver has a unique blood supply network. A primary source of fresh blood to the liver is the arterial inflow from the hepatic artery. But the liver has a secondary blood supply, the portal vein in the abdomen. Being both arterial and venous, these sources of supply function differently. The pulsatile flow of blood from the hepatic artery occurs shortly after systole, like other arterial flow, and the blood supply comes directly from the heart. The inflow of blood from the portal vein occurs later in the heart cycle, and contains blood which has been filtered by the lungs. It has been found that the vascular network which develops to supply blood to a lesion is generally arterial, whereas the blood supplied to normal parenchyma is generally venous. Thus, the relative timing and amount of blood flow from these two sources, if such can be separately distinguished, can lead to effective lesion diagnosis. One technique which can distinguish these different flows of blood is ultrasonic contrast imaging with microbubble contrast agents. In a typical procedure, the subject is infused with either a bolus injection of the contrast agent, or with continuous infusion of the agent. Following a bolus injection, a tumor in the liver will "light up" as it is infused with the arrival of contrast agent from the hepatic arterial blood supply. Normal tissue in the liver lights up at a later time when the bolus of contrast agent enters the liver through the portal vein after passing through the lungs. At this later time the tumor will appear similar to or less bright than the surrounding normal tissue. Since these blood flow conditions occur at different times relative to the start of the contrast agent infusion and are characterized by arterial blood flow at one time and venous flow at another, it would again be desirable to tailor the operation of the ultrasound system so that it is optimized to best detect these different blood flows at the times when they occur during the contrast agent phases.

US 2009/124907 discloses detection of significant liver growths such as HCC lesions during a contrast agent ultrasound exam. A pixel classifier looks for and identifies pixel or voxel regions where early wash-in of contrast occurs and denotes these pixel or voxel locations in a parametric image. The pixel classifier analyzes pixel or voxel values from a sequence of images and identifies suspicious regions in an image by uniquely coding the points in a parametric liver image where early wash-in occurs.

US 2010/172562 discloses setting a liver tumor region for a plurality of ultrasonic image data along a time series acquired by ultrasonically capturing a subject to which a contrast agent has been administered. A TIC generator obtains a time change indicating a time change of the pixel values in the liver tumor region based on the plurality of ultrasonic image data along the time series. A peak-detection section specifies a peak point of the time change and obtains the time and pixel value of that peak point. A first determination section determines the degree of malignancy of the liver tumor based on the time and pixel value of the peak point.

The invention is defined by the independent claims. Advantageous embodiments are provided in the dependent claims.

In accordance with the principles of the present invention an ultrasound system is described which automatically changes system operation during contrast agent wash-in, wash-out to optimize the system for imaging different states of blood and contrast flow during a contrast imaging procedure. A timer tracks the time from the start of infusion or agent arrival at a region of interest, and one or more changes in system operation are invoked during the procedure. Among the operations which may be optimized for different portions of the procedure are ultrasound transmission and receive signal processing, and image processing. The process can be fully automatic so that changes in system operation occur without the need for control manipulation by the user. In an alternative embodiment, the user can decide when the wash-in, wash-out changeover occurs by, for example, observing the peak of a time-intensity curve, and actuate a control which changes multiple transmit/receive parameters at the same time.

In the drawings:
FIGURE 1 illustrates in block diagram form an ultrasonic diagnostic imaging system constructed in accordance with the principles of the present invention.
FIGURE 2 illustrates a method for changing the inclusion of fundamental and harmonic frequency components in image processing during a contrast enhanced imaging procedure.
FIGURE 3 illustrates a method for changing the flow velocity sensitivity and the image noise reduction during a contrast enhanced imaging procedure.
FIGURE 4 illustrates a method for changing the ultrasound pulse transmit frequency during a contrast enhanced imaging procedure.
FIGURE 5 illustrates a method for changing the ultrasound transmit pulse length during a contrast enhanced imaging procedure.
FIGURE 6 illustrates a method for changing the image frame rate of display during a contrast enhanced imaging procedure.

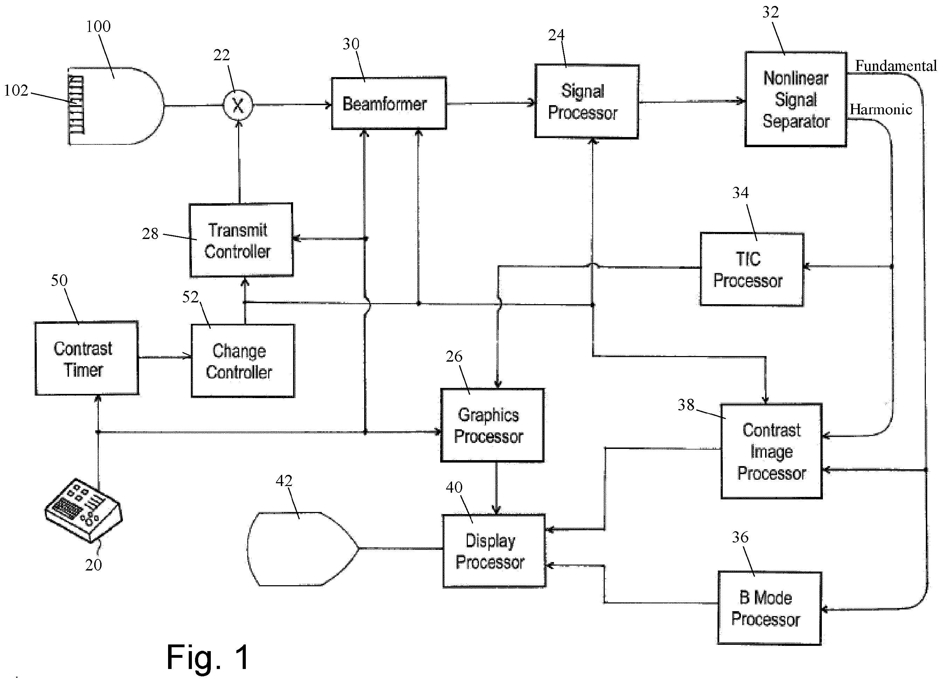

Referring first to FIGURE 1, an ultrasound system constructed in accordance with the principles of the present invention is shown in block diagram form. An ultrasound probe 100 includes an array 102 of ultrasonic transducer elements that transmits ultrasonic pulses and receives ultrasonic echo signals. The array may be a one-dimensional linear or curved array for two-dimensional imaging, or may be a two-dimensional matrix of transducer elements for electronic beam steering and focusing in two or three dimensions. The ultrasonic transducer elements in the array 102 transmit beams of ultrasonic energy by their timed actuation under control of a transmit controller 28, and receive echoes returned in response to each transmission. Echoes from the transmitted ultrasonic energy are received by the transducer elements of the array 102, which generate echo signals that are coupled through a transmit/receive (T/R) switch 22 and digitized by analog to digital converters when the system uses a digital beamformer 30. Analog beamformers may alternatively be used. Control of the ultrasound system and of various control setting for imaging such as probe selection and ROI (region of interest) delineation is effected by user manipulation of the controls of a user control panel 20, such as keys, pushbuttons, and a trackball or computer mouse, which is coupled to various circuitry and processors of the ultrasound system. In the illustrated system the user controls are coupled to provide user input to the transmit controller 28, the beamformer 30, a signal processor 24, and a contrast image processor 38.

The echo signal samples from the transducer elements of the array 102 are delayed and summed by the beamformer 30 to form coherent echo signals along scanline directions for an image. The digital coherent echo signals are then filtered by the signal processor 24, which may also perform noise reduction as by spatial or frequency compounding or persistence processing. The signal processor can also shift the frequency band of the coherent echo signals to a lower or baseband frequency range. The signal processor can be configured as shown in U.S. Patent No. 5,833,613 (Averkiou et al.), for example. When phase information is needed as is the case for Doppler processing, quadrature (I and Q) demodulation may also be performed on the echo signals. In this implementation, the transmit band centered around frequency fₒ and the receiver frequency band are individually controlled so that the beamformer 30 is free to receive a band of frequencies which is different from that of the transmitted band such as one including a harmonic frequency band around frequency 2fₒ.

The beamformed and processed coherent echo signals are coupled to a nonlinear signal separator 32. The nonlinear signal separator can separate second harmonic echo signals with a high pass filter, but preferably it separates harmonic frequencies of echoes returned from contrast agent microbubbles by the pulse inversion technique, in which echo signals resulting from the transmission of multiple, differently phased (inverted) pulses to an image location are additively combined to cancel fundamental signal components and enhance harmonic components, thus producing echo signals in a harmonic band 2fₒ. The harmonic signals can alternatively be separated by amplitude-modulated pulse inversion as described in US Pat. 5,577,505 (Brock-Fisher et al.) The same echo signals are subtractively combined to produce echo signals in a fundamental frequency band fₒ. A preferred pulse inversion technique is described in U.S. patent 6,186,950 (Averkiou et al.) and in U.S. patent 5,706,819 (Hwang et al.) for instance.

Harmonic echo signals from a contrast agent, such as microbubbles, are coupled to a contrast image processor 38. Contrast agents are often used to more clearly delineate blood vessels, or to perform perfusion studies of the microvasculature of tissue as described in US Pat. 6,692,438 (Skyba et al.) for example. In the implementation shown in FIGURE 1, echoes from a contrast agent are used to produce both contrast images and time-intensity curves (TICs) from selected ROIs (individual pixel locations or groups of pixels) in an image field. For parametric contrast images, a 3 by 3 group of pixels is preferred for a pixel area from which to form a time-intensity curve. The contrast image processor produces an anatomical contrast image by amplitude (or envelope) detection of the harmonic frequency echoes from each point in the image field. One way to do this when the echoes are quadrature demodulated is to calculate the signal amplitude at each pixel location in the form of (I²+Q²)^{½}. These contrast intensity signals are mapped to the desired display format by scan conversion which converts samples from R-θ coordinates to Cartesian (x,y) coordinates for display of a spatially defined image.

The fundamental frequency echo signals are coupled to a B mode processor 36 which produces a standard B mode tissue image. The B mode processor performs in the same manner as the contrast image processor, but operates on fundamental frequency echoes. The echo signals are amplitude (envelope) detected and scan converted to produce a spatially delineated image of tissue in the image field. The contrast and B mode images are coupled to a display processor 40 which performs the processing needed to display the images on an image display 42. This may include displaying two images at the same time, side-by-side. It may also comprise overlaying perfusion parameter colors over the B mode images so that perfusion parameters are shown in relation to the tissue structure in which the contrast agent which led to the calculation of the parameters is located.

The harmonic frequency signals returned from contrast agent microbubbles may also be used to measure contrast wash-in and wash-out by forming time-intensity curves of the contrast wash-in and `wash-out. Time-intensity curves are formed by a TIC processor 34 for each point (pixel) in a contrast image. Using the harmonic signal amplitudes acquired during wash-in and wash-out of the contrast agent, curves of contrast intensity at each pixel location are calculated by the TIC processor as described in US pat. pub. no. 2011/0208061 (Chang). The curves are then converted by the TIC processor into a preferred display parameter, such as instantaneous contrast perfusion, peak contrast perfusion, or perfusion rate. A particular time-intensity curve for a chosen location in an ROI can also be graphically displayed by a graphics processor 26. For a parametric perfusion image, the desired parameter for each curve at each pixel location in the ROI is applied to a color map look-up table in the graphics processor 26, where the parameters are converted to corresponding color values. The colors can be those of a range of colors as is done for colorflow imaging, for instance. The resulting map or maps of color parameters are then overlaid over an anatomically corresponding B mode or contrast image, which produces a parametric image of perfusion. The image is coupled to the display processor 40, which displays the parametric image on the image display 42, either alone or side-by-side with a contrast image or a B mode image from the B mode processor.

In accordance with the principles of the present invention, the ultrasound system of FIGURE 1 has a contrast timer 50, which tracks the time elapsed from an injection of contrast agent or from the arrival of a bolus of contrast at an ROI, the latter being automatically detectable by the detection of an increase in harmonic content of echo signals from the ROI. The time measure is coupled to a change controller 52, which institutes one or more changes in system operation at one or more times during contrast agent infusion. Alternatively, the change controller can be actuated by the user upon observation of a pre-determined event such as the peak of the time-intensity curve. In this way, desired changes in system operation, which optimize the system for imaging during pre-determined periods of contrast wash-in, wash-out, are caused to occur by operation of the change controller. The change controller is coupled to the transmit controller 28 to effect desired changes in ultrasound transmit operation; to the beamformer 30 to effect desired changes in beamforming operation; to the signal processor 24 to effect desired changes in signal processing; and to the contrast image processor 38 to effect desired changes in contrast image processing. Through operation of the contrast timer and the change controller, changes in system operation during a contrast imaging procedure are caused to occur automatically without the need for the clinician to divert his attention from the ultrasound images as a contrast agent is applied to and passes through the body in its wash-in and wash-out phases.

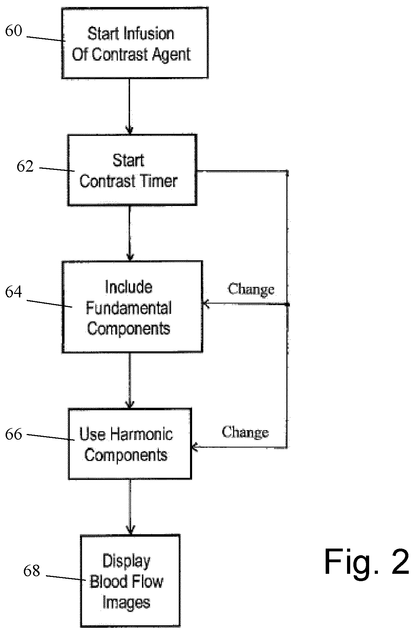

One example of the use of the system of FIGURE 1 to optimize a contrast-enhanced imaging procedure is shown in FIGURE 2. In this example, the blood flow in the vasculature of a developing lesion is being examined. Since the flow velocity in vessels feeding the lesion will be greater than that which infuses the microvasculature of the surrounding normal parenchyma, and the flow of contrast in these larger vessels will occur earlier in the wash-in, wash-out cycle than the parenchyma perfusion, the system is optimized to be sensitive to early stage, high velocity flow. In step 60 of the procedure, an infusion of contrast agent into the subject's bloodstream is started. The clinician presses a button on the control panel 20, which starts the contrast timer 50 in step 62. At the initiation of contrast infusion, the system is set up in step 64 to include fundamental frequency signal components in image processing by the contrast image processor. When the nonlinear signal separation is being performed by the pulse inversion technique, it is important that there be little or no motion in the image field so that the echoes returned from the two transmit events are complementary. But when the echoes are returning from flowing blood, this is not the case due to motion, and the pulse inversion process then is in effect acting as a two-pulse motion detector. This is ideal for detecting the blood flow in vessels of a lesion, and so the system is set up at this time to apply these motion-sensitive signals in the fundamental frequency band to the contrast image processor for imaging this blood flow motion. The contrast image processor can also be conditioned during this time to operate as a Doppler detector, producing measures of the flow velocity in the vessels for imaging. A velocity image is produced by the contrast image processor 38, which overlays a tissue image produced by the B mode processor 36 to depict blood flow velocity in blood vessels, which is displayed to the clinician in step 68. In these ways, the ultrasound system is conditioned at this time to be sensitive to relatively high velocity blood flow in blood vessels feeding a lesion.

As the infusion of contrast continues the contrast agent will begin to flow in the microvasculature of the parenchyma as well as the vessels of the lesion. To optimally view both areas of contrast, the contrast timer 50 triggers the change controller 52 at a later time in the wash-in, wash-out cycle to change the image processing. The change command to the contrast image processor 38 ends the use of fundamental signals by the contrast image processor in step 64, which now produces contrast images with harmonic signals from the nonlinear signal separator 32 as shown by step 66. The parenchyma will light up with harmonic contrast signals at this time. Harmonic contrast images are now displayed to the clinician in step 68, and both the vascular flow and the parenchymal perfusion can now be observed by the clinician. This change in system optimization occurs automatically at a predetermined time in the contrast infusion process, without the need to distract the clinician to manipulate any control panel controls as the continually changing contrast images are being observed.

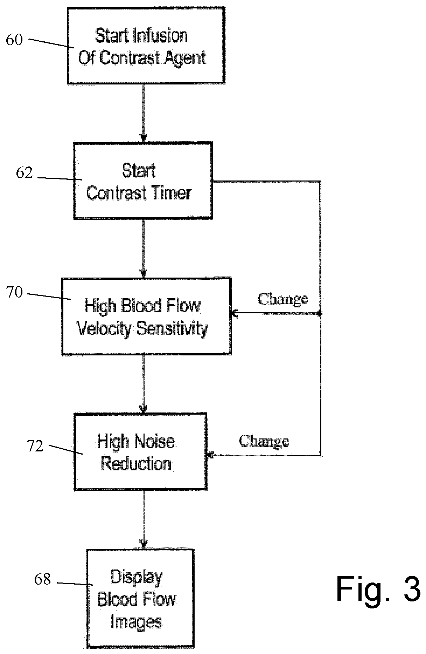

Another example of the use of the system of FIGURE 1 to optimize a contrast-enhanced imaging procedure is shown in FIGURE 3. Steps 60, 62 and 68 have been previously described. In this second example, the ultrasound system is initially conditioned to be sensitive for high blood flow velocity detection, as shown in step 70. In particular, image noise reduction efforts are minimized, particularly those that use temporal processing for noise reduction. Thus, frame-to-frame changes in high velocity flow such as the arrival of the contrast agent in larger vessels can be observed without temporal blurring. When the contrast agent has arrived in all areas of the region of interest later in the wash-in, wash-out cycle and has begun to stabilize, the change controller 52 initiates a change in step 72 to greater noise reduction processing so that the relatively stationary microbubbles in the parenchyma can be clearly observed. This may be done, for example, by commanding the transmit controller 28, the beamformer 30, and the signal processor 24 to spatially compound multiple images to reduce speckle noise in the images. Another example is commanding the initiation of persistence processing of successive images by the signal processor. In this later stage of the infusion process, the system is optimized for greater clarity of the parenchymal perfusion.

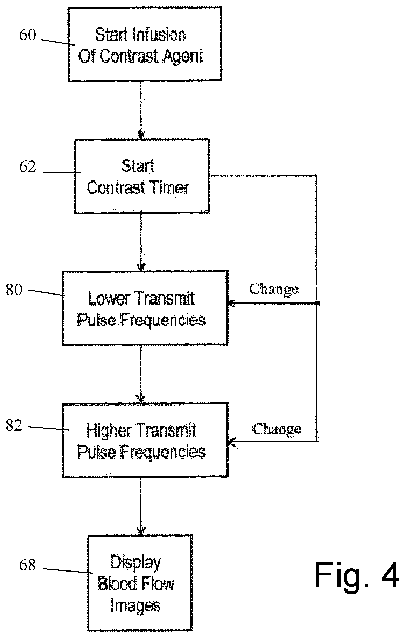

Another example of the use of the system of FIGURE 1 to optimize a contrast-enhanced imaging procedure is shown in FIGURE 4. In this example, the ultrasound system initially begins imaging with lower transmit pulse frequencies as shown in step 80. The use of lower pulse frequencies by the transmit controller 28 and transducer array 102 causes greater resonance and hence greater echo signal returns from larger microbubbles of the contrast agent. Larger microbubbles would be expected to flow in the larger blood vessels of a lesion, for example. In liver diagnosis, larger microbubbles would be expected in arterial blood flow which comes directly from the heart, as compared with venous flow during the portal stage where the blood has been filtered by the lungs and primarily only smaller microbubbles remain. The optimization of the lower transmit frequencies enables the arterial flow to be more readily distinguished from the portal flow in a contrast-enhanced liver exam.

When the contrast timer has reached a predetermined time of the contrast procedure, the change controller 52 causes the transmit controller 28 to change to the use of higher transmit pulse frequencies, as shown in step 82. The higher pulse frequencies will resonate most strongly with smaller microbubbles of the contrast agent. This will, for instance, optimize the ultrasound system for imaging the contrast agent in the parenchyma, where the microvasculature is too fine to allow passage of larger microbubbles. It will also optimize the system toward more optimal imaging of portal flows during a liver exam, where the blood flow will be largely populated by smaller microbubbles. The change controller, in this example, is changing the transmit signal processing of the system.

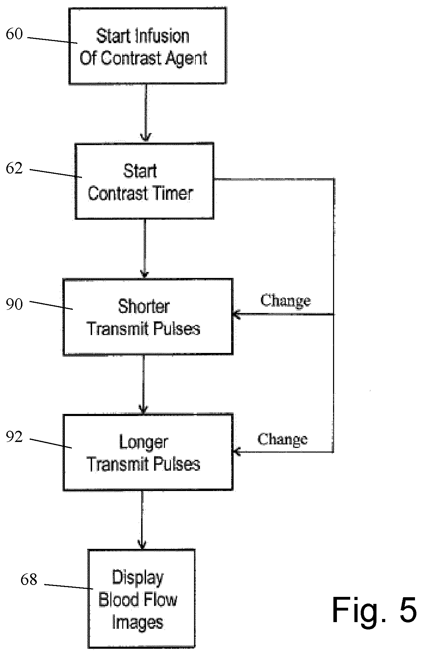

Another example of the use of the system of FIGURE 1 to optimize a contrast-enhanced imaging procedure, again through transmit control, is shown in FIGURE 5. In this example, the ultrasound system is initially conditioned to transmit shorter pulses (fewer cycles) during the initial portion of the wash-in, wash-out cycle as shown in step 90. The use of short transmit pulses is well suited to the initial arrival of the contrast bolus, when contrast flow velocities in an ROI are relatively high and velocity detection is being performed by the contrast image processor. Later in the wash-out phase of the cycle, after the parenchyma has become perfused and flow velocities are lower, the sensitivity for low flow detection is improved by the use of longer transmit pulses as shown by step 92. The change controller 52 causes a change by the transmit controller 28 to the use of longer transmit pulses during the latter portion of the wash-in, wash-out cycle for greater flow sensitivity in the images.

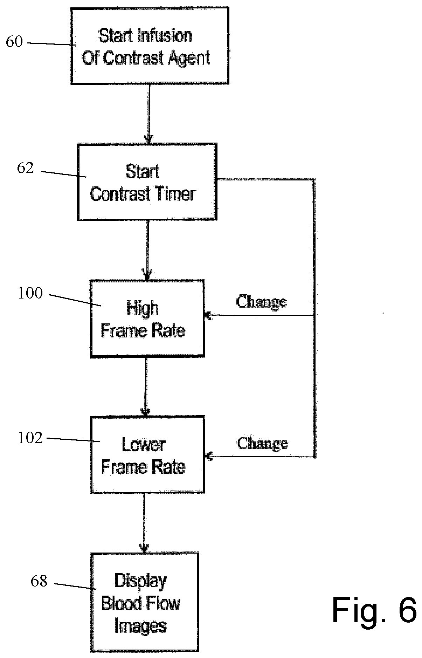

Another example of the use of the system of FIGURE 1 to optimize a contrast-enhanced imaging procedure is shown in FIGURE 6. In this example, the ultrasound system is initially conditioned to optimally image rapid changes in contrast flow by using a higher frame rate of display during the wash-in phase, as shown in step 100. This enables clearer imaging of the rapid build-up of contrast agent in the ROI as the bolus of contrast arrives in the blood vessels of the ROI. It can also provide clearer images of the arterial flow during liver contrast imaging procedures. During the wash-out phase of the procedure, there is no longer a rapid build-up of contrast, but a slow decline in the concentration of contrast in the ROI. For this phase, the change controller 52 commands the transmit, receive, and signal processing components of the ultrasound system to change to a lower frame rate of display as shown in step 102, which is well suited to more slowly changing conditions in the ROI. For example, the lower frame rate could be traded-off for better spatial resolution (by increasing the line density) or longer bubble lasting times (by reducing the number of transmit events per second).

Other variations and modifications will readily occur to those skilled in the art. The different changes shown in the example can be combined, for example. For instance, the frame rate change of the method of FIGURE 6 can also be combined with change of the transmit pulse frequency or length as illustrated in FIGURES 4 and 5, and the initiation of more noise reduction as shown in FIGURE 3. The changes invoked by the change controller do not have to be instantaneous, but can be slower transitional changes occurring gradually over time. In implementations having a TIC processor 34, it is possible to replace the contrast timer with a time-intensity curve produced by the TIC processor, with changes in system operation occurring in relation to the time, duration or flow events demarcated by the time-intensity curve. The changes in system operation can thus occur in response to events of the wash-in, wash-out cycle, such as the occurrence of the peak of contrast wash-in. Other changes to the receive signal path which can be invoked to improve diagnostic performance include gain/TGC increases during washout to improve signal sensitivity; dynamic range reduction during washout to improve signal conspicuity; and image processing algorithm changes during washout to take account of the diffused nature of the bubble response in the late phase as compared to the single-bubble response commonly encountered during the arterial phase.

It should be noted that an ultrasound system which acquires contrast echo signal data and processed it to form an optimized contrast image, and in particular the component structure of the ultrasound system of FIGURE 1, may be implemented in hardware, software or a combination thereof. The various embodiments and/or components of an ultrasound system, for example, the modules, or components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system which stores previously acquired contrast images. The computer or processor may also include a memory. The memory devices, such as a memory storing predetermined change times for the change controller 52, may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including the acquisition of contrast data and the calculation of time-intensity curves and parameters described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

## Claims

1. An ultrasonic diagnostic imaging system which produces contrast-enhanced images comprising:
a transducer array probe (100) adapted to transmit ultrasound pulses and receive echo signals;
a transmit controller (28) coupled to the transducer array probe;
a beamformer (30) coupled to the transducer array probe and adapted to produce coherent echo signals;
a signal processor (24) coupled to the beamformer;
a contrast image processor (38) coupled to the signal processor and adapted to produce contrast-enhanced ultrasound images;
an image display (42) adapted to display the contrast-enhanced ultrasound images;
a contrast timer (50) adapted to track time from the start of contrast agent infusion or contrast agent arrival at a region of interest; the ultrasound diagnostic imaging system **characterized by**
a change controller (52), responsive to the contrast timer, which is adapted to cause a change in operation of one or more of the transmit controller, the beamformer, the signal processor, or the contrast image processor to optimize the ultrasonic diagnostic imaging system for imaging during different portions of a cycle of contrast agent wash-in, wash-out.

2. The ultrasonic diagnostic imaging system of Claim 1, wherein the change controller is further adapted to cause a change in use of fundamental frequency components by the contrast image processor.

3. The ultrasonic diagnostic imaging system of Claim 2, wherein the change controller is further adapted to cause a change from use of fundamental frequency components to use of harmonic frequency components by the contrast image processor at a predetermined time during a cycle of contrast agent wash-in, wash-out.

4. The ultrasonic diagnostic imaging system of Claim 1, wherein the change controller is further adapted to cause a change in blood flow velocity sensitivity by the contrast image processor at a predetermined time during a cycle of contrast agent wash-in, wash-out.

5. The ultrasonic diagnostic imaging system of Claim 1, wherein the change controller is further adapted to cause a change in the amount of noise reduction at a predetermined time during a cycle of contrast agent wash-in, wash-out.

6. The ultrasonic diagnostic imaging system of Claim 1, wherein the change controller is further adapted to cause a change in transmit frequency controlled by the transmit controller, and
optionally wherein the change controller is further adapted to cause a change in transmit pulse frequency of the transducer array probe from lower frequencies to higher frequencies during a cycle of contrast agent wash-in, wash-out.

7. The ultrasonic diagnostic imaging system of Claim 1, wherein the change controller is further adapted to cause a change in transmit pulse length controlled by the transmit controller, and
optionally wherein the change controller is further adapted to cause a change in transmit pulse length of the transducer array probe from shorter pulses to longer pulses during a cycle of contrast agent wash-in, wash-out.

8. The ultrasonic diagnostic imaging system of Claim 1, wherein the change controller is further adapted to cause a change in image frame rate during a cycle of contrast agent wash-in, wash-out, and
optionally wherein the change controller is further adapted to cause a change in image frame rate from a high frame rate to a lower frame rate during a cycle of contrast agent wash-in, wash-out.

9. The ultrasonic diagnostic imaging system of any of Claims 1 to 8, further comprising a nonlinear signal separator, coupled to receive ultrasound echo signals acquired in the presence of contrast agent flow, and configured to produce harmonic echo signal data, and
optionally wherein the nonlinear signal separator is further configured to produce one or both of harmonic echo signal data or fundamental echo signal data.

10. The ultrasonic diagnostic imaging system of any of Claims 1 to 9, further comprising a time-intensity curve processor, responsive to echo signal data from contrast flow, which is configured to produce time-intensity curves of contrast flow in a region of interest.

11. The ultrasonic diagnostic imaging system of Claim 10, wherein the contrast timer further comprises the time-intensity curve processor,
wherein the change controller is further responsive to a time-intensity curve to cause a change in operation of the ultrasonic system.

12. A method for changing an ultrasound diagnostic imaging system operation during a contrast enhanced imaging procedure, comprising the steps of:
receiving at an ultrasound diagnostic imaging system an input of an ultrasound image signal for a period of time;
receiving at the ultrasound diagnostic imaging system an input of a contrast agent infusion start time;
measuring with a contrast timer an elapsed time from the start time; **characterized by**
changing, based on the measuring step, an operation of the ultrasound diagnostic imaging system, wherein the changing step comprises a change in operation of one or more of an ultrasound diagnostic imaging system transmit controller, a beamformer, a signal processor, and a contrast image processor to optimize the ultrasonic diagnostic imaging system for imaging during different portions of a cycle of contrast agent wash-in, wash-out.

13. A computer program product embodied in a non-volatile computer readable medium and providing instructions to change an ultrasound diagnostic imaging system operation during a contrast enhanced imaging procedure, the instructions comprising the steps of:
receiving at an ultrasound diagnostic imaging system an input of an ultrasound image signal for a period of time;
receiving at the ultrasound diagnostic imaging system an input of a contrast agent infusion start time;
measuring with a contrast timer an elapsed time from the start time; **characterised in that** the instructions further comprises
changing, based on the measuring step, an operation of the ultrasound diagnostic imaging system, wherein the changing step comprises a change in operation of one or more of an ultrasound diagnostic imaging system transmit controller, a beamformer, a signal processor, and a contrast image processor to optimize the ultrasonic diagnostic imaging system for imaging during different portions of a cycle of contrast agent wash-in, wash-out.

## Patentansprüche

1. Ultraschall-Diagnosebildgebungssystem, das kontrastverstärkte Bilder erzeugt, umfassend:
eine Wandlergruppierungssonde (100), die zum Senden von Ultraschallimpulsen und Empfangen von Echosignalen geeignet ist;
eine Sendesteuereinheit (28), die mit der Wandlergruppierungssonde gekoppelt ist;
einen Strahlformer (30), der mit der Wandlergruppierungssonde gekoppelt und zum Erzeugen kohärenter Echosignale geeignet ist;
einen Signalprozessor (24), der mit dem Strahlformer gekoppelt ist;
einen Kontrastbildprozessor (38), der mit dem Signalprozessor gekoppelt und zum Erzeugen kontrastverstärkter Ultraschallbilder geeignet ist;
eine Bildanzeige (42), die zum Anzeigen der kontrastverstärkten Ultraschallbilder geeignet ist;
einen Kontrast-Zeitgeber (50), der dazu geeignet ist, die Zeit ab dem Beginn der Kontrastmittelinfusion oder dem Eintreffen des Kontrastmittels in einem Bereich von Interesse zu verfolgen; wobei das Ultraschall-Diagnosebildgebungssystem durch Folgendes gekennzeichnet ist:
eine Änderungssteuereinheit (52), reagierend auf den Kontrast-Zeitgeber, die geeignet ist, eine Änderung im Betrieb eines oder mehrerer von der Sendesteuereinheit, dem Strahlformer, dem Signalprozessor oder dem Kontrastbildprozessor zu bewirken, um das Ultraschall-Diagnosebildgebungssystem für die Bildgebung während unterschiedlicher Abschnitte eines Zyklus von Kontrastmittel-Einwaschen, -Auswaschen zu optimieren.

2. Ultraschall-Diagnosebildgebungssystem nach Anspruch 1, wobei die Änderungssteuereinheit weiter geeignet ist, eine Änderung bei der Verwendung von Grundfrequenzkomponenten durch den Kontrastbildprozessor zu bewirken.

3. Ultraschall-Diagnosebildgebungssystem nach Anspruch 2, wobei die Änderungssteuereinheit weiter geeignet ist, zu einem vorbestimmten Zeitpunkt während eines Zyklus des Kontrastmittel-Einwaschens, -Auswaschens eine Änderung von der Verwendung von Grundfrequenzkomponenten zur Verwendung von harmonischen Frequenzkomponenten durch den Kontrastbildprozessor zu bewirken.

4. Ultraschall-Diagnosebildgebungssystem nach Anspruch 1, wobei die Änderungssteuereinheit weiter geeignet ist, zu einem vorbestimmten Zeitpunkt während eines Zyklus von Kontrastmittel-Einwaschens, -Auswaschens eine Änderung bei der Blutflussgeschwindigkeitsempfindlichkeit durch den Kontrastbildprozessor zu bewirken.

5. Ultraschall-Diagnosebildgebungssystem nach Anspruch 1, wobei die Änderungssteuereinheit weiter geeignet ist, zu einem vorbestimmten Zeitpunkt während eines Zyklus des Kontrastmittel-Einwaschens, -Auswaschens eine Änderung beim Rauschunterdrückungsausmaß zu bewirken.

6. Ultraschall-Diagnosebildgebungssystem nach Anspruch 1, wobei die Änderungssteuereinheit weiter geeignet ist, eine Änderung bei der Sendefrequenz zu bewirken, die durch die Sendesteuereinheit gesteuert wird, und
optional wobei die Änderungssteuereinheit weiter geeignet ist, während eines Zyklus des Kontrastmittel-Einwaschens, -Auswaschens eine Änderung der Sendeimpulsfrequenz der Wandlergruppierungssonde von niedrigeren Frequenzen zu höheren Frequenzen zu bewirken.

7. Ultraschall-Diagnosebildgebungssystem nach Anspruch 1, wobei die Änderungssteuereinheit weiter geeignet ist, eine Änderung bei der Sendeimpulslänge zu bewirken, die durch die Sendesteuereinheit gesteuert wird, und
optional wobei die Änderungssteuereinheit weiter geeignet ist, während eines Zyklus des Kontrastmittel-Einwaschens, -Auswaschens eine Änderung bei der Sendeimpulslänge der Wandlergruppierungssonde von kürzeren Impulsen zu längeren Impulsen zu bewirken.

8. Ultraschall-Diagnosebildgebungssystem nach Anspruch 1, wobei die Änderungssteuereinheit weiter geeignet ist, während eines Zyklus des Kontrastmittel-Einwaschens, -Auswaschens eine Änderung bei einer Bildframerate zu bewirken, und
optional wobei die Änderungssteuereinheit weiter geeignet ist, während eines Zyklus des Kontrastmittel-Einwaschens, -Auswaschens eine Änderung bei der Bildframerate von einer hohen Framerate zu einer niedrigeren Framerate zu bewirken.

9. Ultraschall-Diagnosebildgebungssystem nach einem der Ansprüche 1 bis 8, weiter umfassend einen nichtlinearen Signalseparator, der zum Empfangen von Ultraschall-Echosignalen, die in der Gegenwart eines Kontrastmittelflusses erfasst werden, gekoppelt ist und zum Erzeugen von harmonischen Echosignaldaten konfiguriert ist, und
optional wobei der nichtlineare Signalseparator weiter dafür konfiguriert ist, eines oder beide von harmonischen Echosignaldaten oder fundamentalen Echosignaldaten zu erzeugen.

10. Ultraschall-Diagnosebildgebungssystem nach einem der Ansprüche 1 bis 9, weiter umfassend einen Zeit-Intensitätskurvenprozessor, reagierend auf Echosignaldaten von dem Kontrastmittelfluss, der dafür konfiguriert ist, Zeit-Intensitätskurven des Kontrastmittelflusses in einem Bereich von Interesse zu erzeugen.

11. Ultraschall-Diagnosebildgebungssystem nach Anspruch 10, wobei der Kontrast-Zeitgeber weiter den Zeit-Intensitätskurvenprozessor umfasst,
wobei der Änderungssteuereinheit weiter auf eine Zeit-Intensitätskurve reagiert, um eine Änderung im Betrieb des Ultraschallsystems zu bewirken.

12. Verfahren zur Änderung des Betriebs eines Ultraschall-Diagnosebildgebungssystems während eines kontrastverstärkten Bildgebungsvorgangs, umfassend die folgenden Schritte:
Empfangen einer Eingabe eines Ultraschallbildsignals an einem Ultraschall-Diagnosebildgebungssystem über einen Zeitraum;
Empfangen einer Eingabe einer Kontrastmittelinfusion-Startzeit am Ultraschall-Diagnosebildgebungssystem;
Messen einer verstrichenen Zeit ab dem Startzeitpunkt mit einem Kontrast-Zeitgeber; **gekennzeichnet durch**
Ändern eines Betriebs des Ultraschall-Diagnosebildgebungssystems, basierend auf dem Messschritt, wobei der Änderungsschritt eine Änderung beim Betrieb eines oder mehrerer von einer Sendesteuereinheit des Ultraschall-Diagnosebildgebungssystems, einem Strahlformer, einem Signalprozessor und einem Kontrastbildprozessor umfasst, um das Ultraschall-Diagnosebildgebungssystem für die Bildgebung während unterschiedlicher Abschnitte eines Zyklus von Kontrastmittel-Einwaschen, -Auswaschen zu optimieren.

13. Computerprogrammprodukt, das in einem nichtflüchtigen computerlesbaren Medium verkörpert ist und Anweisungen zur Änderung des Betriebs eines Ultraschall-Diagnosebildgebungssystems während eines kontrastverstärkten Bildgebungsvorgangs bereitstellt, wobei die Anweisungen die folgenden Schritte umfassen:
Empfangen einer Eingabe eines Ultraschallbildsignals an einem Ultraschall-Diagnosebildgebungssystem über einen Zeitraum;
Empfangen einer Eingabe einer Kontrastmittelinfusion-Startzeit am Ultraschall-Diagnosebildgebungssystem;
Messen einer verstrichenen Zeit ab dem Startzeitpunkt mit einem Kontrast-Zeitgeber; **dadurch gekennzeichnet, dass** die Anweisungen weiter Folgendes umfassen:
Ändern eines Betriebs des Ultraschall-Diagnosebildgebungssystems, basierend auf dem Messschritt, wobei der Änderungsschritt eine Änderung beim Betrieb eines oder mehrerer von einer Sendesteuereinheit des Ultraschall-Diagnosebildgebungssystems, einem Strahlformer, einem Signalprozessor und einem Kontrastbildprozessor umfasst, um das Ultraschall-Diagnosebildgebungssystem für die Bildgebung während unterschiedlicher Abschnitte eines Zyklus von Kontrastmittel-Einwaschen, -Auswaschen zu optimieren.

## Revendications

1. Système d'imagerie diagnostique ultrasonore qui produit des images à contraste amélioré comprenant :
une sonde à réseau de transducteurs (100) adaptée pour transmettre des impulsions ultrasonores et recevoir des signaux d'écho ;
un dispositif de commande d'émission (28) couplé à la sonde à réseau de transducteurs ;
un dispositif de formation de faisceaux (30) couplé à la sonde à réseau de transducteurs et adapté pour produire des signaux d'écho cohérents ;
un processeur de signaux (24) couplé au dispositif de formation de faisceaux ;
un processeur d'images de contraste (38) couplé au processeur de signaux et adapté pour produire des images ultrasonores à contraste amélioré ;
un dispositif d'affichage d'images (42) adapté pour afficher les images ultrasonores à contraste amélioré ;
un minuteur de contraste (50) adapté pour suivre le temps écoulé depuis le début de la perfusion de l'agent de contraste ou l'arrivée de l'agent de contraste dans une zone d'intérêt ; le système d'imagerie diagnostique ultrasonore étant **caractérisé par**
un dispositif de commande de modification (52), sensible au minuteur de contraste, qui est adapté pour provoquer une modification du fonctionnement d'un ou plusieurs éléments parmi le dispositif de commande d'émission, le dispositif de formation de faisceaux, le processeur de signaux ou le processeur d'images de contraste afin d'optimiser le système d'imagerie diagnostique ultrasonore pour l'imagerie pendant différentes phases d'un cycle d'injection et d'élimination de l'agent de contraste.

2. Système d'imagerie diagnostique ultrasonore selon la revendication 1, dans lequel le dispositif de commande de modification est adapté en outre pour provoquer une modification dans l'utilisation de composantes de fréquences fondamentales par le processeur d'images de contraste.

3. Système d'imagerie diagnostique ultrasonore selon la revendication 2, dans lequel le dispositif de commande de modification est adapté en outre pour provoquer une modification de l'utilisation de composantes de fréquences fondamentales à l'utilisation de composantes de fréquences harmoniques par le processeur d'images de contraste à un moment prédéterminé au cours d'un cycle d'injection et d'élimination de l'agent de contraste.

4. Système d'imagerie diagnostique ultrasonore selon la revendication 1, dans lequel le dispositif de commande de modification est adapté en outre pour provoquer une modification de sensibilité de vitesse d'écoulement sanguin par le processeur d'images de contraste à un moment prédéterminé au cours d'un cycle d'injection et d'élimination de l'agent de contraste.

5. Système d'imagerie diagnostique ultrasonore selon la revendication 1, dans lequel le dispositif de commande de modification est adapté en outre pour provoquer une modification de niveau de réduction du bruit à un moment prédéterminé au cours d'un cycle d'injection et d'élimination de l'agent de contraste.

6. Système d'imagerie diagnostique ultrasonore selon la revendication 1, dans lequel le dispositif de commande de modification est adapté en outre pour provoquer une modification de fréquence d'émission commandée par le dispositif de commande d'émission, et
facultativement, dans lequel le dispositif de commande de modification est adapté pour provoquer une modification de fréquence d'impulsions d'émission de la sonde à réseau de transducteurs, passant de fréquences plus basses à des fréquences plus élevées au cours d'un cycle d'injection et d'élimination de l'agent de contraste.

7. Système d'imagerie diagnostique ultrasonore selon la revendication 1, dans lequel le dispositif de commande de modification est adapté en outre pour provoquer une modification de longueur d'impulsions d'émission commandée par le dispositif de commande d'émission, et
facultativement, dans lequel le dispositif de commande de modification est adapté en outre pour provoquer une modification de la longueur d'impulsions d'émission de la sonde à réseau de transducteurs, passant d'impulsions plus courtes à des impulsions plus longues au cours d'un cycle d'injection et d'élimination de l'agent de contraste.

8. Système d'imagerie diagnostique ultrasonore selon la revendication 1, dans lequel le dispositif de commande de modification est adapté en outre pour provoquer une modification de la fréquence de trames d'images au cours d'un cycle d'injection et d'élimination de l'agent de contraste, et
facultativement, dans lequel le dispositif de commande de modification est adapté en outre pour provoquer une modification de trames d'images, passant d'une fréquence de trames d'images élevée à une fréquence de trames d'images plus faible au cours d'un cycle d'injection et d'élimination de l'agent de contraste.

9. Système d'imagerie diagnostique ultrasonore selon l'une quelconque des revendications 1 à 8, comprenant en outre un séparateur de signaux non linéaires, couplé de manière à recevoir des signaux d'écho ultrasonore acquis en présence d'un écoulement de l'agent de contraste et configuré pour produire des données de signaux d'échos harmoniques, et
facultativement, dans lequel le séparateur de signaux non linéaires est configuré en outre pour produire un ou les deux parmi les données de signaux d'échos harmoniques ou les données de signaux d'échos fondamentaux.

10. Système d'imagerie diagnostique ultrasonore selon l'une quelconque des revendications 1 à 9, comprenant en outre un processeur de courbes temps-intensité, sensible aux données de signaux d'écho provenant flux de contraste, qui est configuré pour produire des courbes temps-intensité du flux de contraste dans une zone d'intérêt.

11. Système d'imagerie diagnostique ultrasonore selon la revendication 10, dans lequel le minuteur de contraste comprend en outre le processeur de courbes temps-intensité,
dans lequel le dispositif de commande de modification est sensible en outre à une courbe temps-intensité pour provoquer une modification du fonctionnement du système ultrasonore.

12. Procédé de modification d'un fonctionnement du système d'imagerie diagnostique ultrasonore au cours d'une procédure d'imagerie à contraste amélioré, comprenant les étapes de :
réception, au niveau d'un système d'imagerie diagnostique ultrasonore, d'une entrée d'un signal d'images ultrasonores pendant une période donnée ;
réception, au niveau du système d'imagerie diagnostique ultrasonore, d'une entrée de l'heure de début de perfusion de l'agent de contraste ;
mesure, à l'aide d'un minuteur de contraste, d'un temps écoulé depuis l'heure de début ; **caractérisé par**
la modification, en fonction de l'étape de mesure, d'un fonctionnement du système d'imagerie diagnostique ultrasonore, dans lequel l'étape de modification comprend une modification du fonctionnement d'un ou plusieurs éléments parmi un dispositif de commande démission du système d'imagerie diagnostique ultrasonore, un dispositif de formation de faisceaux, un processeur de signaux et un processeur d'images de contraste afin d'optimiser le système d'imagerie diagnostique ultrasonore pour l'imagerie pendant différentes phases d'un cycle d'injection et d'élimination de l'agent de contraste.

13. Produit de programme informatique intégré à un support non volatil lisible par ordinateur et fournissant des instructions pour modifier un fonctionnement d'un système d'imagerie diagnostique ultrasonore au cours d'une procédure d'imagerie à contraste amélioré, ces instructions comprenant les étapes de :
réception, au niveau d'un système d'imagerie diagnostique ultrasonore, d'une entrée d'un signal d'images ultrasonores pendant une période donnée ;
réception, au niveau du système d'imagerie diagnostique ultrasonore, d'une entrée de l'heure de début de perfusion de l'agent de contraste ;
mesure, à l'aide d'un minuteur de contraste, d'un temps écoulé depuis l'heure de début ; **caractérisé en ce que** les instructions comprennent en outre
la modification, en fonction de l'étape de mesure, d'un fonctionnement du système d'imagerie diagnostique ultrasonore, dans lequel l'étape de modification comprend une modification du fonctionnement d'un ou plusieurs éléments parmi un dispositif de commande démission du système d'imagerie diagnostique ultrasonore, un dispositif de formation de faisceaux, un processeur de signaux et un processeur d'images de contraste afin d'optimiser le système d'imagerie diagnostique ultrasonore pour l'imagerie pendant différentes phases d'un cycle d'injection et d'élimination de l'agent de contraste.
